# EUROPEAN PATENT APPLICATION

(11) **EP 3 933 847 A1**
(43) Date of publication of application: **05.01.2022**
(21) Application number: 19917106.7
(22) Date of filing: 16.08.2019
(51) Int. Cl.: G16H 30/20

(54) **VRDS 4D MEDICAL IMAGE MULTI-DEVICE AI INTERCONNECTED DISPLAY METHOD AND PRODUCT**

(30) Priority: 28.02.2019 CN 201910152226
(71) Applicant: VR Doctor Medical Technology (Shenzhen) Co., Ltd., Shenzhen, Guangdong, 518035 (CN)
(72) Inventor: LEE, David Wei, Shenzhen, Guangdong 518035 (CN); LEE, Stewart Ping, Shenzhen, Guangdong 518035 (CN)
(74) Representative: Dargiewicz, Joanna
(86) International application number: PCT/CN2019/101162
(87) International publication number: WO 2020/173055

(57) **Abstract**

A method and a product for multi-device AI linkage displaying of a VRDS 4D medical image, the method includes: extracting first partial image data from a target 4D image data according to preset raw spatial attitude information and display screen parameters of a first display device, and extracting second partial image data from the target 4D image data according to the raw spatial attitude information and display screen parameters of a second display device; and displaying the first partial image data on the first display device and displaying the second partial image data on the second display device; judging whether to enable a linkage display function when receiving first spatial attitude information and second spatial attitude information; if so, selecting the spatial attitude information of a display device with high display priority as reference spatial attitude information; adjusting image data displayed by a display device with low display priority.

## Description

### TECHNICAL FIELD

This application relates to the field of medical imaging apparatus, and in particular, to a method and a product for multi-device AI linkage displaying of a VRDS 4D medical image.

### BACKGROUND

In current, doctors still use watching to read continuous two-dimensional slice scanned images, so as to perform judging and analyzing on the pathological tissue of the patients such as tumors. However, the two-dimensional slice scanned image can not present the spatial structure characteristics of pathological tissue, which affects the diagnosis of the doctors on the diseases. With rapid development of medical imaging technology, people put forward new demands for medical imaging.

### SUMMARY

Embodiments of this application provide a method and a product for multi-device AI linkage displaying of a VRDS 4D medical image, so as to improve the real-time and intelligence of a medical imaging apparatus in performing multi-device medical image linkage display.

In a first aspect, embodiments of this application provides a method for multi-device AI linkage displaying of a VRDS 4D medical image, which is applied to a server of a medical imaging apparatus, wherein the medical imaging apparatus includes the server, a first display device and a second display device, and the server is connected with the first display device and the second display device; the method includes:
acquiring a target four-dimensional (4D) image data; extracting a first partial image data from the target 4D image data according to preset raw spatial attitude information and display screen parameters of the first display device; and extracting a second partial image data from the target 4D image data according to the raw spatial attitude information and display screen parameters of the second display device, wherein the target 4D image data includes image data of an inner space and an outer contour of a displayed target object;
displaying the first partial image data on the first display device and displaying the second partial image data on the second display device;
   judging whether to enable a linkage display function when receiving first spatial attitude information from the first display device and second spatial attitude information from the second display device, wherein the first spatial attitude information is spatial attitude control information collected by the first display device and the second spatial attitude information is spatial attitude control information collected by the second display device, and the first spatial attitude information is different from the raw spatial attitude information;
   if so, selecting spatial attitude information of a display device with high display priority as reference spatial attitude information;
   adjusting image data displayed by a display device with low display priority according to the reference spatial attitude information and a display screen parameters of the display device with low display priority, and outputting linkage prompt information based on the display device with high display priority on the display device with low display priority.

In a second aspect, embodiments of this application provides a apparatus for multi-device AI linkage displaying of a VRDS 4D medical image, which is applied to a server of a medical imaging apparatus, wherein the medical imaging apparatus includes the server, a first display device and a second display device, and the server is connected with the first display device and the second display device; the apparatus for multi-device AI linkage displaying of a VRDS 4D medical image includes a processing unit and a communication unit, wherein,
the processing unit is configured to: acquire target 4D image data, extract first partial image data from the target 4D image data according to preset raw spatial attitude information and display screen parameters of the first display device, and extract second partial image data from the target 4D image data according to the raw spatial attitude information and display screen parameters of the second display device, wherein the target 4D image data includes image data of an inner space and an outer contour of a displayed target object; display the first partial image data on the first display device and display the second partial image data on the second display device; and judge whether to enable a linkage display function when receiving first spatial attitude information from the first display device and second spatial attitude information from the second display device, wherein the first spatial attitude information is spatial attitude control information collected by the first display device and the second spatial attitude information is spatial attitude control information collected by the second display device, and the first spatial attitude information is different from the raw spatial attitude information; and if so, select spatial attitude information of a display device with high display priority as reference spatial attitude information; and adjust image data displayed by a display device with low display priority according to the reference spatial attitude information and the display screen parameters of the display device with low display priority, and output linkage prompt information based on the display device with high display priority on the display device with low display priority.

In a third aspect, embodiments of this application provide a medical imaging apparatus, the apparatus includes a processor, a memory, a communication interface, and one or more programs, wherein the one or more programs are stored in the memory and configured to be executed by the processor, and the programs include instructions for executing the steps in any method of the first aspect of the embodiment of this application.

In a fourth aspect, embodiments of this application provide a computer readable storage medium, wherein the computer readable storage medium stores a computer program for electronic data exchange, wherein the computer program causes a computer to execute some or all of the steps described in any methods of the first aspect of the embodiment of this application.

In a fifth aspect, embodiments of this application provide a computer program product, wherein the computer program product includes a non-transitory computer-readable storage medium in which a computer program is stored, the computer program is operable to cause the computer to execute some or all of the steps described in any methods of the first aspect of the embodiment of this application. The computer program product can be a software installation package.

As can be seen that in the embodiment of this application, first, the medical imaging apparatus acquires the target 4D image data, extracts first partial image data from a target 4D image data according to preset raw spatial attitude information and display screen parameters of a first display device, and extracts second partial image data from the target 4D image data according to the raw spatial attitude information and display screen parameters of a second display device, second, displays the first partial image data on the first display device and displays the second partial image data on the second display device; and then, judges whether to enable a linkage display function when receiving the first spatial attitude information from the first display device and the second spatial attitude information from the second display device, if so, selects the spatial attitude information of a display device with high display priority as reference spatial attitude information, and finally, adjusts image data displayed by a display device with low display priority according to the reference spatial attitude information and the display screen parameters of the display device with low display priority, and outputs linkage prompt information based on the display device with high display priority on the display device with low display priority. It can be seen that the medical imaging apparatus of this application can perform real-time linkage display control of multi-device for 4D images, and can handle the linkage display control conflicts of multi-device based on priority mechanism, rationally arrange active and passive display devices, thereby improving the real-time and intelligence of a medical imaging apparatus in performing linkage displaying of multi-device medical images.

### BRIEF DESCRIPTION OF THE DRAWINGS

In order to describe the technical solutions in the embodiments of this application or in the prior art more clearly, the following briefly describes the accompanying drawings required for describing the embodiments or the prior art, apparently, the accompanying drawings in the following description only show some embodiments of this application, and the ordinary skilled person in the art may still derive other drawings from these accompanying drawings without creative efforts.
Fig. 1 is a schematic structural diagram of an intelligent analyzing and processing system based on VRDS 4D medical images provided by an embodiment of this application;
Fig. 2 is a schematic flowchart of a method for multi-device AI linkage displaying of a VRDS 4D medical image provided by an embodiment of this application;
Fig. 3 is a schematic structural diagram of a medical imaging apparatus provided by an embodiment of this application;
Fig. 4 is a block diagram of functional units composition of an apparatus for multi-device AI linkage displaying of a VRDS 4D medical image provided by an embodiment of this application.

### DETAILED DESCRIPTION OF PREFERRED EMBODIMENTS

In order to make person in the art better understand the solution of this application, the following clearly and completely describes the technical solutions in the embodiments of this application with reference to the accompanying drawings in the embodiments of this application, apparently, the described embodiments are only some but not all of the embodiments of this application. All other embodiments obtained by the ordinary skill person in the art based on the embodiments of this application without creative efforts shall fall within the protection scope of this application.

The terms "first", "second", etc. in the specification and claims of this application and the above drawings are used to distinguish different objects, but not to describe a specific order. Furthermore, that term "including" and "have" and any variations thereof are intended to cover non-exclusive inclusion. For example, a process, method, system, product or device including a series of steps or units is not limited to the listed steps or units, but optionally further includes steps or units not listed, or optionally further includes other steps or units inherent to these processes, methods, products or devices.

Reference to an "embodiment" herein means that a particular feature, structure or characteristic described in connection with an embodiment may be included in at least one embodiment of this application. The appearances of the phrase in various places in the specification are not necessarily all referring to the same embodiment, nor are separate or alternative embodiments mutually exclusive of other embodiments. It is explicitly and implicitly to be understood by the person skilled in the art that the described embodiment may be combined with other embodiments.

The medical imaging apparatuses related to the embodiments of this application refer to various instruments that use various different media as information carriers to reproduce the internal structure of the human body as images, their image information has a spatial and temporal distribution corresponding relationship with the actual structure of the human body. "DICOM data" refers to the raw image file data collected by medical device and reflecting the internal structure features of human body, and can include information such as Computed Tomography (CT), Nuclear Magnetic Resonance (MRI), Diffusion Tensor Imaging (DTI), Positron Emission Computed Tomography (PET), "image source" refers to Texture 2D/3D image volume data generated by parsing the raw DICOM data. "VRDS" refers to Virtual Reality Doctor system.

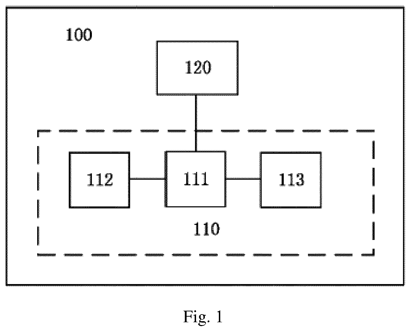

Referring to Fig. 1, Fig. 1 is a schematic structural diagram of an intelligent analyzing and processing system 100 based on VRDS 4D medical images provided by an embodiment of this application, the system 100 includes a medical imaging apparatus 110 and a network database 120, wherein the medical imaging apparatus 110 may include a server 111, a first display device 112 and a second display device 113, and the server 111 is connected with the first display device 112 and the second display device 113, the medical imaging apparatus 110 is configured to perform multi-device linkage displaying on four-dimensional medical images based on the raw DICOM data and the multi-device AI linkage display algorithm based on VRDS 4D medical images presented in the embodiment of this application (the four-dimensional medical image specifically refers to the medical image including the internal spatial structure features and the external spatial structure features of the displayed tissue, the internal spatial structure features refer to slice data inside the tissues are not lost, that is, medical imaging apparatuses can present the internal constructions of tissues such as target organs and blood vessels, and the external spatial structure characteristics refer to the environmental features between tissues, including spatial position characteristics (including intersection, spacing and fusion) between tissues, such as edge structure characteristics of the intersection position between kidney and artery, etc.), the medical imaging apparatus 110 can be further configured to edit the image source data to form the transfer function results of the four-dimensional human body image, which can include the transfer function results of the surface of the internal organs of the human body and the tissue structure inside the internal organs of the human body, as well as the transfer function results of the cube spatial, such as the number, coordinates, colors, transparency and other information of the cube editing boxes and arc editing arrays required by the transfer function. The network database 120 can be, for example, a cloud server, etc., the network database 120 is configured to store the image source generated by parsing the raw DICOM data and the transfer function result of the four-dimensional human body image edited by the medical imaging apparatus 110, the image source can come from a plurality of medical imaging apparatuses 110 to achieve the interactive diagnosis of a plurality of doctors.

The following describes a method for multi-device AI linkage displaying of VRDS 4D medical image medical images in detail.

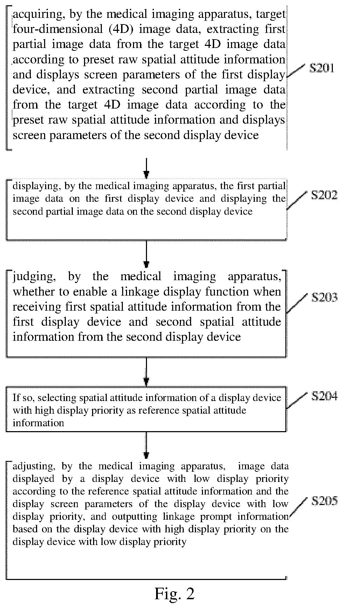

Referring to Fig. 2, Fig. 2 is a schematic flowchart of a method for multi-device AI linkage displaying of a VRDS 4D medical image provided by an embodiment of this application, which is applied to the medical imaging apparatus as described in Fig. 1; as shown in the Figure, the method for multi-device AI linkage displaying of a VRDS 4D medical image includes:
S201, acquiring, by a medical imaging apparatus, a target four-dimensional 4D image data, extracting first partial image data from the target 4D image data according to preset raw spatial attitude information and displays screen parameters of the first display device, and extracting second partial image data from the target 4D image data according to the raw spatial attitude information and displays screen parameters of the second display device, wherein the target 4D image data includes image data of an inner space and an outer contour of a displayed target object.

Wherein, the 4D image data refers to the data related to the presentation of four-dimensional images, and the raw spatial attitude information refers to the initial spatial attitude of the target object, that is, the spatial attitude of the target object initially displayed by the medical imaging apparatus, here, the raw spatial attitude information of the first and second display devices is the same.

Here, the display screen parameters refer to parameters such as screen material, color number, contrast, brightness, etc.

Wherein, the display screen parameters of the first display device and the second display device may be completely different or partially the same. As far as the parameters of the same display screen are concerned, they can be the same or different and taking the screen material in the display screen parameters as an example, the display screen can be LED display screen, STN display screen, UFB display screen, TFD display screen, TFT display screen, IPS display screen and OLED display screen, wherein the display screen of the first display device and the display screen of the second display device can be the same, for example, a display screen of the first display device and a display screen of the second display device are both TFD display screens, which may be different, for example, a display screen of the first display device is a UFB display screen, and a display screen of the second display device is a TFD display screen.

Wherein, the first partial image data and the second partial image data can be completely different or partially intersected, which is not particularly limited. For example, the first partial image data may be image data of the inner space of the target object displayed in the target 4D image data, and the second partial image data may be image data of the outer contour of the target object displayed in the target 4D image data.

In a specific implementation, the medical imaging apparatus can determine the spatial constraint condition of the pixel points on the display screen according to the parameters of the display screen, and determine the image data of the target object to be displayed in the initial spatial attitude information state according to the spatial constraint condition, for the head-mounted device VR display device, that is the image data observed from the initial human eye perspective.

In this possible example, the implementation way of acquiring, by the medical imaging apparatus, the target four-dimensional 4D image data can be: acquiring, by the medical imaging apparatus, the target four-dimensional 4D image data from a database in a network environment; the implementation way of acquiring, by the medical imaging apparatus, the target four-dimensional 4D image data can also be: acquiring, by the medical imaging apparatus, target four-dimensional 4D image data from other terminal devices; the implementation way of acquiring, by the medical imaging apparatus, the target four-dimensional 4D image data can also be acquiring a scanned image for the target user, wherein the scanned image includes any one of the following images: CT image, MRI image, DTI image and PET-CT image, then determining a bitmap (BMP) data source according to the image, and then generating target medical image data according to the BMP data source, and finally, screening, by the medical imaging apparatus, enhanced data with quality scores larger than preset scores from the target medical image data as 4D image data, wherein the quality scores can be comprehensively evaluated from the following dimensions, such as average gradient, information entropy, visual information fidelity, peak signal-to-noise ratio (PSNR), structural similarity index measurement (SSIM), mean square error (MSE), etc., and specific reference can be made to common image quality score algorithm in the image field, which will not be repeated here; the medical imaging apparatus can also acquire the target 4D image data by other implementation ways, which is not specifically limited.

It can be seen that in this example, the medical imaging apparatus can extract the 4D image data corresponding to the display screen parameters of the display device from the 4D image data according to the display screen parameters of the display device, thus improving the pertinence of displaying the 4D image data by the display device.

S202, displaying, by the medical imaging apparatus, the first partial image data on the first display device and the second partial image data on the second display device.

Wherein, the displaying, by the medical imaging apparatus, the first partial image data on the first display device and the second partial image data on the second display device can be displaying, by the medical imaging apparatus, the first partial image data on the first display device and the second partial image data on the second display device at the same time.

In this possible example, the first partial image data may be image data of the inner space of the target object displayed in the target 4D image data, and the second partial image data may be image data of the outer contour of the target object displayed in the target 4D image data; and the implementation way of the displaying, by the medical imaging apparatus, the first partial image data on the first display device and the second partial image data on the second display device can be displaying the image data of the inner space of the target object displayed in the target 4D image data on the first display device, and displaying the image data of the outer contour of the target object displayed in the target 4D image data on the first display device.

It can be seen that in this example, the medical imaging apparatus can extract corresponding 4D image data from the 4D image data according to the display screen parameters of the display device, and perform the corresponding display, and finally achieve different split screen display of the data in the target 4D image data.

S203, judging, by the medical imaging apparatus, whether to enable a linkage display function when receiving first spatial attitude information from the first display device and second spatial attitude information from the second display device, wherein the first spatial attitude information is spatial attitude control information collected by the first display device and the second spatial attitude information is spatial attitude control information collected by the second display device, and the first spatial attitude information is different from the raw spatial attitude information.

Wherein, the spatial attitude control information refers to the information that controls the orientation of the target object.

Wherein, the spatial attitude control information collected by the display device may be the information for controlling of spatial attitude that collected by the display device and generated by the operational actions of users. Wherein, operation actions refer to the operation control of the four-dimensional human body image by the user through external intake devices of the medical imaging apparatus, such as a mouse and a keyboard, so as to achieve human-computer interaction, the operation actions include at least one of the following: (1) changing the color and/or transparency of a specific organ/tissue, (2) positioning and scaling the view, (3) rotating the view to achieve multi-view 360-degree observation of the four-dimensional human image, (4) "entering" inside the organ of the human body to observe the internal construction, and the shearing effect rendering in real time, and (5) moving the view up and down.

In this possible example, the implementation way of the judging whether to enable the linkage display function can be detecting whether a linkage display switch of the server is turned on; if it is detected that the linkage display switch is turned on, determining to enable the linkage display function; and if it is detected that the linkage display switch is turned off, determining not to enable the linkage display function.

Wherein, the linkage display switch can be a physical switch or a virtual function button, the physical switch can be set on the server or set exclusively on the first or second display device, and the virtual function button can be in the display interface of the first or second display device.

It can be seen that in this example, the medical imaging apparatus supports the active setting of the linkage display function, which improves the convenience and flexibility of use.

In this possible example, the implementation way of the judging whether to enable the linkage display function can also be detecting whether a difference between the first spatial attitude information and the second spatial attitude information is within a preset range; if so, determining to enable the linkage display function; if not, determining not to enable the linkage display function.

Wherein, the specific implementation way for the medical imaging apparatus to detect whether a difference between the first spatial attitude information and the second spatial attitude information is within a preset range can be: acquiring, by the medical imaging apparatus, the first space attitude information and the second spatial attitude information; calculating the difference between the first spatial attitude information and the second spatial attitude information; comparing the difference with a preset range; if the difference falls within the preset range, determining to enable the linkage display function; and if the difference is not within the preset range, determining not to enable the linkage display function.

It can be seen that in this example, the medical imaging apparatus judges whether to enable the linkage display function according to the spatial attitude information of a plurality of display devices, which improves the intelligence of the medical imaging device for linkage display.

In this possible example, the implementation way of the judging whether to enable the linkage display function can be: detecting whether the first display device and the second display device are used by a same user through a camera; if so, determining to enable the linkage display function; if not, determining not to enable the linkage display function.

Wherein, the implementation way for the medical imaging apparatus detecting of whether the first display device and the second display device are used by the same user through the camera can be, acquiring, by the medical imaging apparatus, one or more image frames within the coverage area of the camera through the camera, and the image frames include the first display device, the second display device and a user; recognizing, by the medical imaging apparatus, whether the user in the image frame is one according to the image frame; if so, determining that the first display device and the second display device are used by the same user; if not, determining whether it is used by the same user according to the distance between a plurality of users and the first display device and the second display device.

It can be seen that in this example, the medical imaging apparatus can detect that the current use scene is an independent user use scene through the camera; and automatically enabling the linkage display function at this time is more in line with the potential needs of users to locate specific images, and improves the intelligence of the medical imaging apparatus for linkage display.

S204, if so, selecting spatial attitude information of a display device with high display priority as reference spatial attitude information.

In this possible example, selecting the spatial attitude information of the display device with high display priority as the reference spatial attitude information may be selecting the spatial attitude information of the display device with high display priority set by the user as the reference spatial pose information; selecting the spatial attitude information of display devices with high display priority as reference spatial attitude information can also be obtaining the security level of display devices and using the spatial attitude information of display devices with high security level as reference spatial attitude information; selecting spatial attitude information of display devices with high display priority as reference spatial attitude information may also be obtaining historical data of display devices with high display priority and using spatial attitude information of display devices with high frequency as reference spatial attitude information.

It can be seen that in this example, the medical imaging apparatus selects the corresponding spatial attitude information as the reference spatial attitude information through the priority of the display device, which improves the intelligence of linkage display of the medical imaging apparatus.

S205, adjusting, by the medical imaging apparatus, image data displayed by a display device with low display priority according to the reference spatial attitude information and the display screen parameters of the display device with low display priority, and outputting linkage prompt information based on the display device with high display priority on the display device with low display priority.

In this possible example, the implementation way for the medical imaging apparatus adjusting the image data displayed by the display device with low display priority according to the reference spatial attitude information and the display screen parameters of the display device with low display priority can be: extracting, by the medical imaging apparatus, third partial image data from the target 4D image data according to the reference spatial attitude information and the display screen parameters of the display device with low display priority; updating image data displayed by the display device with low display priority to the third partial image data.

Wherein, the third partial image data in the 4D image data may be completely different or partially the same as the first partial image data and the second partial image data in the target 4D image data.

It can be seen that in this example, the medical imaging apparatus can extract additional image data for updating and displaying according to the reference spatial attitude information and the display screen parameters of the display device with low display priority, which improves the intelligence of the medical imaging apparatus for linkage display.

In this possible example, the target 4D image data includes a plurality of pieces of image data of the target object, and each image data includes pixel information and spatial position information of corresponding pixel points, wherein the spatial position information is configured to represent a spatial position attribute of the pixel points in a 4D pixel spatial structure of the target object, and the 4D pixel spatial structure corresponds to a real spatial structure of the target object; extracting, by the medical imaging apparatus, the third partial image data from the target 4D image data according to the reference spatial attitude information and the display screen parameters of the display device with low display priority, including: determining by the medical imaging apparatus, a spatial screening range of the image data according to the reference spatial attitude information and the display screen parameters of the display device with low display priority; screening out a plurality of pieces of image data belonging to the spatial screening range from the plurality of pieces of image data, wherein the plurality of pieces of image data are the third partial image data.

It can be seen that in this example, medical imaging apparatus adjusts image data displayed by the display device with low display priority according to the reference spatial attitude information and the display screen parameters of the display device with low display priority, and outputs linkage prompt information based on the display device with high display priority on the display device with low display priority, thereby improving the effect of multi-device linkage display.

In one possible embodiment, the first display device includes a virtual reality (VR) display device, and the second display device includes a personal computer (PC) display device; the target 4D image data includes image data of a kidney site of the target user, the kidney site includes kidney, tumor and blood vessel, the kidney has a blood supply relationship with the tumor, the blood vessel includes a first blood vessel adjacent to the tumor and a second blood vessel connected to the tumor, and the first blood vessel and the second blood vessel are in different positions and have an intersection position; the method further includes: if it is judged that the linkage display function is not enabled, displaying the second blood vessel on the PC display device while the first blood vessel is displayed on the VR display device; extracting image data of the intersection position of the first blood vessel and the second blood vessel when a selection instruction for the first blood vessel and the second blood vessel is detected; and displaying images including the image data of the intersection position on the VR display device and the PC display device respectively.

It can be seen that in this example, the medical imaging apparatus can display different images in different VR display devices according to the user operation variationaly, and intelligently screen blood vessel intersection position and display images, thus improving the intelligence and convenience of image display.

As can be seen that in the embodiment of this application, first, the medical imaging apparatus acquires the target 4D image data, extracts first partial image data from the target 4D image data according to preset raw spatial attitude information and display screen parameters of the first display device, and extracts second partial image data from the target 4D image data according to the raw spatial attitude information and display screen parameters of the second display device; second, displays the first partial image data on the first display device and displays the second partial image data on the second display device; and then, judges whether to enable a linkage display function when receiving the first spatial attitude information from the first display device and the second spatial attitude information from the second display device, if so, selects the spatial attitude information of a display device with high display priority as reference spatial attitude information; and finally, adjusts image data displayed by a display device with low display priority according to the reference spatial attitude information and the display screen parameters of the display device with low display priority, and outputs linkage prompt information based on the display device with high display priority on the display device with low display priority. It can be seen that the medical imaging apparatus of this application can perform real-time linkage display control of multi-device for 4D images, and can handle the linkage display control conflicts of multi-device based on priority mechanism, rationally arrange active and passive display device, thereby improving the real-time and intelligence of a medical imaging apparatus in performing linkage displaying of multi-device medical images.

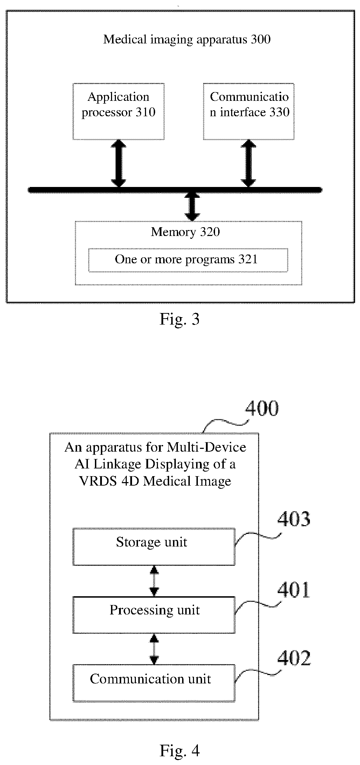

Consistent with the embodiments shown in Fig. 2, referring to Fig. 3, Fig. 3 is a schematic structural diagram of a medical imaging apparatus 300 provided by an embodiment of this application, as shown in the figure, the medical imaging apparatus 300 includes a processor 310, a memory 320, a communication interface 330 and one or more programs 321, wherein the one or more programs 321 are stored in the memory 320 and configured to be executed by the processor 310. T he one or more programs 321 include instructions for executing the following steps: acquiring target four-dimensional 4D image data; extracting first partial image data from the target 4D image data according to preset raw spatial attitude information and display screen parameters of the first display device, and extracting second partial image data from the target 4D image data according to the raw spatial attitude information and display screen parameters of the second display device, wherein the target 4D image data includes image data of an inner space and an outer contour of a displayed target object; and judging whether to enable a linkage display function when receiving first spatial attitude information from the first display device and second spatial attitude information from the second display device, wherein the first spatial attitude information is spatial attitude control information collected by the first display device and the second spatial attitude information is spatial attitude control information collected by the second display device, and the first spatial attitude information is different from the raw spatial attitude information; and if so, selecting spatial attitude information of a display device with high display priority as reference spatial attitude information; and adjusting image data displayed by a display device with low display priority according to the reference spatial attitude information and the display screen parameters of the display device with low display priority; and outputting linkage prompt information based on the display device with high display priority on the display device with low display priority.

As can be seen that in the embodiment of this application, first, the medical imaging apparatus acquires the target 4D image data, extracts first partial image data from a target 4D image data according to preset raw spatial attitude information and display screen parameters of a first display device, and extracts second partial image data from the target 4D image data according to the raw spatial attitude information and display screen parameters of a second display device; second, displays the first partial image data on the first display device and displays the second partial image data on the second display device; and then, judges whether to enable a linkage display function when receiving the first spatial attitude information from the first display device and the second spatial attitude information from the second display device, if so, selects the spatial attitude information of a display device with high display priority as reference spatial attitude information; and finally, adjusts image data displayed by a display device with low display priority according to the reference spatial attitude information and the display screen parameters of the display device with low display priority; and outputs linkage prompt information based on the display device with high display priority on the display device with low display priority. It can be seen that the medical imaging apparatus of this application can perform real-time linkage display control of multi-device for 4D images, and can handle the linkage display control conflicts of multi-device based on priority mechanism, rationally arrange active and passive display device, thereby improving the real-time and intelligence of linkage displaying of a medical imaging apparatus in performing multi-device medical images.

In one possible example, in the aspect of the judging of whether to enable the linkage display function, the instructions in the program are specifically configured to perform the following operation: detecting whether a linkage display switch of the server is turned on; if it is detected that the linkage display switch is turned on, determining to enable the linkage display function; and if it is detected that the linkage display switch is turned off, determining not to enable the linkage display function.

In one possible example, in the aspect of the judging of whether to enable the linkage display function, the instructions in the program are specifically configured to perform the following operation: detecting whether a difference between the first spatial attitude information and the second spatial attitude information is within a preset range; if so, determining to enable the linkage display function; if not, determining not to enable the linkage display function.

In one possible example, in the aspect of the judging of whether to enable the linkage display function, the instructions in the program are specifically configured to perform the following operation: detecting whether the first display device and the second display device are used by the same user through a camera; if so, determining to enable the linkage display function; if not, determining not to enable the linkage display function.

In one possible example, in the aspect of the adjusting of the image data displayed by the display device with low display priority according to the reference spatial attitude information and the display screen parameters of the display device with low display priority, the instructions in the program are specifically configured to perform the following operation: extracting third partial image data from the target 4D image data according to the reference spatial attitude information and the display screen parameters of the display device with low display priority; updating image data displayed by the display device with low display priority to the third partial image data.

In one possible example, the target 4D image data includes a plurality of pieces of image data of the target object, and each image data includes pixel information and spatial position information of corresponding pixel points, wherein the spatial position information is configured to represent spatial a position attribute of the pixel points in a 4D pixel spatial structure of the target object, and the 4D pixel spatial structure corresponds to a real spatial structure of the target object. In the aspect of the extracting of the third partial image data from the target 4D image data according to the reference spatial attitude information and the display screen parameters of the display device with low display priority, the instructions in the program are specifically configured to perform the following operation: determining a spatial screening range of the image data according to the reference spatial attitude information and the display screen parameters of the display device with low display priority; screening out a plurality of pieces of image data belonging to the spatial screening range from the plurality of pieces of image data, wherein the plurality of pieces of image data are the third partial image data.

In one possible example, the first display device includes a virtual reality (VR) display device, and the second display device includes a personal computer (PC) display device; the target 4D image data includes image data of a kidney site of the target user, the kidney site includes kidney, tumor and blood vessel, the kidney has a blood supply relationship with the tumor, the blood vessel includes a first blood vessel adjacent to the tumor and a second blood vessel connected to the tumor, and the first blood vessel and the second blood vessel are in different positions and have an intersection position. The one or more programs 321 include instructions for executing the following steps: if it is judged that the linkage display function is not enabled, displaying the second blood vessel on the PC display device while the first blood vessel is displayed on the VR display device; extracting image data of the intersection position of the first blood vessel and the second blood vessel when a selection instruction for the first blood vessel and the second blood vessel is detected; and displaying images including the image data of the intersection position on the VR display device and the PC display device respectively.

The above mainly introduces the solution of the embodiment of this application from the perspective of the execution process on the method side. It can be understood that in order to achieve the above functions, the medical imaging apparatus includes corresponding hardware structures and/or software modules for performing various functions. It should be easy for the skilled person in the art aware that, in combination with the units and algorithmic steps of the examples described in the embodiments provided herein, this application can be implemented in the form of hardware or a combination of hardware and computer software. Whether the functions are performed by hardware or computer software driving hardware depends on particular applications and design constraint conditions of the technical solutions. A person skilled in the art may use different methods to implement the described functions for each particular application, but it should not be considered that the implementation goes beyond the scope of this application.

The embodiment of this application can divide the medical imaging apparatus into functional units according to the above method example, for example, individual functional unit can be divided corresponding to individual function, or two or more functions can be integrated into one processing unit. The integrated units can be implemented in the form of hardware, and can also be implemented in the form of a software functional unit. It should be noted that the division of units in the embodiment of this application is schematic, which is only a logical function division, and there may be another division mode in actual implementation.

Fig. 4 is a block diagram of functional units composition of an apparatus 400 multi-device AI linkage displaying of a VRDS 4D medical image involved in the embodiment of this application. The apparatus 400 for multi-device AI linkage displaying of a VRDS 4D medical image is applied to a medical imaging apparatus, the apparatus 400 for multi-device AI linkage displaying of a VRDS 4D medical image includes a processing unit 401 and a communication unit 402, wherein,
the processing unit 401 is configured to: acquire target 4D image data, extracting first partial image data from the target 4D image data according to preset raw spatial attitude information and display screen parameters of the first display device, and extract second partial image data from the target 4D image data according to the raw spatial attitude information and display screen parameters of the second display device, wherein the target 4D image data includes image data of an inner space and an outer contour of a displayed target object; and judge whether to enable a linkage display function when receiving first spatial attitude information from the first display device and second spatial attitude information from the second display device, wherein the first spatial attitude information is spatial attitude control information collected by the first display device and the second spatial attitude information is spatial attitude control information collected by the second display device, and the first spatial attitude information is different from the raw spatial attitude information; and if so, select spatial attitude information of a display device with high display priority as reference spatial attitude information; and adjust image data displayed by a display device with low display priority according to the reference spatial attitude information and the display screen parameters of the display device with low display priority, and output linkage prompt information based on the display device with high display priority on the display device with low display priority.

The apparatus 400 for multi-device AI linkage displaying of a VRDS 4D medical image further includes a storage unit 403, wherein the processing unit 401 can be a processor, the communication unit 402 can be a transceiver, and the storage unit can be a memory.

As can be seen that in the embodiment of this application, first, the medical imaging apparatus acquires the target 4D image data, extracts first partial image data from the target 4D image data according to preset raw spatial attitude information and display screen parameters of the first display device, and extracts second partial image data from the target 4D image data according to the raw spatial attitude information and display screen parameters of the second display device; second, displays the first partial image data on the first display device and displays the second partial image data on the second display device; and then, judges whether to enable a linkage display function when receiving the first spatial attitude information from the first display device and the second spatial attitude information from the second display device, if so, selects the spatial attitude information of a display device with high display priority as reference spatial attitude information; and finally, adjusts image data displayed by a display device with low display priority according to the reference spatial attitude information and the display screen parameters of the display device with low display priority, and outputs linkage prompt information based on the display device with high display priority on the display device with low display priority. It can be seen that the medical imaging apparatus of this application can perform real-time linkage display control of multi-device for 4D images, and can handle the linkage display control conflicts of multi-device based on priority mechanism, rationally arrange active and passive display device, thereby improving the real-time and intelligence of a medical imaging apparatus in performing linkage displaying of multi-device medical images.

In one possible example, in the aspect of the judging of whether to enable the linkage display function, the processing unit 401 is specifically configured to: detect whether a linkage display switch of the server is turned on; if it is detected that the linkage display switch is turned on, determine to enable the linkage display function; and if it is detected that the linkage display switch is turned off, determine not to enable the linkage display function.

In one possible example, in the aspect of the judging of whether to enable the linkage display function, the processing unit 401 is specifically configured to: detect whether a difference between the first spatial attitude information and the second spatial attitude information is within a preset range; if so, determine to enable the linkage display function; if not, determine not to enable the linkage display function.

In one possible example, in the aspect of the judging of whether to enable the linkage display function, the processing unit 401 is specifically configured to: detect whether the first display device and the second display device are used by the same user through a camera; if so, determine to enable the linkage display function; if not, determine not to enable the linkage display function.

In one possible example, in the aspect of the adjusting of the image data displayed by the display device with low display priority according to the reference spatial attitude information and the display screen parameters of the display device with low display priority, the processing unit 401 is specifically configured to: extract third partial image data from the target 4D image data according to the reference spatial attitude information and the display screen parameters of the display device with low display priority; update the image data displayed by the display device with low display priority to the third partial image data.

In one possible example, the target 4D image data includes a plurality of pieces of image data of the target object, and each image data includes pixel information and spatial position information of corresponding pixel points, wherein the spatial position information is configured to represent spatial a position attribute of the pixel points in a 4D pixel spatial structure of the target object, and the 4D pixel spatial structure corresponds to a real spatial structure of the target object. In the aspect of the extracting of the third partial image data from the target 4D image data according to the reference spatial attitude information and the display screen parameters of the display device with low display priority, the processing unit 401 is specifically configured to: determine a spatial screening range of the image data according to the reference spatial attitude information and the display screen parameters of the display device with low display priority; screen out a plurality of pieces of image data belonging to the spatial screening range from the plurality of pieces of image data, wherein the plurality of pieces of image data are the third partial image data.

In one possible example, the first display device includes a virtual reality (VR) display device, and the second display device includes a personal computer (PC) display device; the target 4D image data includes image data of a kidney site of the target user, the kidney site includes kidney, tumor and blood vessel, the kidney has a blood supply relationship with the tumor, the blood vessel includes a first blood vessel adjacent to the tumor and a second blood vessel connected to the tumor, and the first blood vessel and the second blood vessel are in different positions and have an intersection position. The processing unit 401 is further configured to: if it is judged that the linkage display function is not enabled, display the second blood vessel on the PC display device while the first blood vessel is displayed on the VR display device; extract image data of the intersection position of the first blood vessel and the second blood vessel when a selection instruction for the first blood vessel and the second blood vessel is detected; and display images including the image data of the intersection position on the VR display device and the PC display device respectively.

Embodiments of this application further provide a computer storage medium, wherein the computer storage medium stores a computer program for electronic data exchange, the computer program causes a computer to execute part or all of the steps of any method as recorded in the above method embodiment, and the computer includes a medical imaging apparatus.

Embodiments of this application further provide a computer program product, the above computer program product includes a non-transitory computer-readable storage medium in which a computer program is stored, the computer program is operable to cause a computer to execute part or all of the steps of any method as recorded in the above method embodiments. The computer program product can be a software installation package, and the computer includes a medical imaging apparatus.

It should be noted that, for brevity of description, the foregoing method embodiments are described as a series of movement combinations. However, the skill person in the art should learn that this application is not limited by the movement sequence, because according to this application, some steps can be performed in other order or simultaneously. Secondly, it also should be known by those skilled in the art that the embodiments described in the specification are preferred embodiments and the involved actions and modules are not the must of the present application necessarily.

In the above embodiments, the descriptions of individual embodiment have their own emphasis, for those parts that are not detailed in one embodiment, please refer to the relevant descriptions of other embodiments.

In the several embodiments provided in this application, it should be understood that the disclosed apparatus may be implemented in other manners. For example, the described apparatus embodiment is merely an example. For example, the above unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, or other forms.

The above units described as separate parts may or may not be physically separate, and parts displayed as units may or may not be physical units, may be located in one position, or may be distributed on a plurality of network units. It can select some or all of units to achieve the objective of the solution of the present embodiment based on actual requirements.

In addition, functional units in the embodiments of this application may be integrated into one processing unit, or each of the units may exist alone physically, or two or more units are integrated into one unit. The integrated units can be implemented in the form of hardware, and can also be implemented in the form of a software functional unit.

When the above integrated units are implemented in the form of a software functional unit and sold or used as an independent product, the functions may be stored in a computer readable memory. Based on such an understanding, the technical solutions of this application essentially, or the part contributing to the prior art, all or some of the technical solutions may be implemented in a form of a software product. The computer software product is stored in a memory, and includes several instructions for instructing a computer device (which may be a personal computer, a server, or a network device) to perform all or some of the steps of the above methods described in the embodiments of this application. The foregoing memory includes: any medium that can store program code, such as a USB flash disk, a read-only memory (ROM), a random access memory (RAM), a removable hard disk, a magnetic disk, or an optical disc.

The ordinary skill person in the art may understand that all or some of the steps in various methods of the above embodiments can be completed by instructing related hardware through programs, which can be stored in a computer readable memory, which can include: flash disks, Read-Only Memory (ROM), random access memory (RAM) disks or optical disks, etc.

The embodiments of this application are described in detail above, and the principles and implementation way of this application are explained by specific examples. The above embodiments are only used to help understand the method and its core ideas of this application; at the same time, according to the idea of this application, there will be some changes in the specific implementation way and application scope for the ordinary skill person in the art, to sum up, the contents of this specification should not be construed as limitations of this application.

## Claims

1. A method for multi-device AI linkage displaying of a Virtual Reality Doctor system (VRDS) 4D medical image, **characterized in that** the method is applied to a server of a medical imaging apparatus, wherein the medical imaging apparatus comprises the server, a first display device and a second display device, the server is connected to the first display device and the second display device; and the method comprises:
acquiring a target four-dimensional 4D image data, extracting a first partial image data from the target 4D image data according to preset raw spatial attitude information and display screen parameters of the first display device, and extracting a second partial image data from the target 4D image data according to the raw spatial attitude information and display screen parameters of the second display device, wherein the target 4D image data comprises image data of an inner space and an outer contour of a displayed target object;
displaying the first partial image data on the first display device and displaying the second partial image data on the second display device;
judging whether to enable a linkage display function when receiving first spatial attitude information from the first display device and second spatial attitude information from the second display device, wherein the first spatial attitude information is spatial attitude control information collected by the first display device and the second spatial attitude information is spatial attitude control information collected by the second display device, and the first spatial attitude information is different from the raw spatial attitude information;
if so, selecting spatial attitude information of a display device with high display priority as reference spatial attitude information;
adjusting image data displayed by a display device with low display priority according to the reference spatial attitude information and the display screen parameters of the display device with low display priority, and outputting linkage prompt information based on the display device with high display priority on the display device with low display priority.

2. The method according to claim 1, **characterized in that** the step of judging whether to enable the linkage display function comprises: detecting whether a linkage display switch of the server is turned on;
if the linkage display switch is detected to be turned on, then determining to enable the linkage display function;
and if the linkage display switch is detected to be turned off, then determining not to enable the linkage display function.

3. The method according to claim 1, **characterized in that** the step of judging whether to enable the linkage display function comprises: detecting whether a difference between the first spatial attitude information and the second spatial attitude information is within a preset range; if so, determining to enable the linkage display function;
if not, determining not to enable the linkage display function.

4. The method according to claim 3, **characterized in that** the step of detecting whether the difference between the first spatial attitude information and the second spatial attitude information is within a preset range comprises:
acquiring the first spatial attitude information and the second spatial attitude information;
calculating the difference between the first spatial attitude information and the second spatial attitude information;
comparing the difference with the preset range;
if the difference falls within the preset range, determining to enable the linkage display function;
and if the difference is not within the preset range, determining not to enable the linkage display function.

5. The method according to claim 1, **characterized in that** the step of judging whether to enable the linkage display function comprises: detecting whether the first display device and the second display device are used by a same user through a camera; if so, determining to enable the linkage display function;
if not, determining not to enable the linkage display function.

6. The method according to claim 4, **characterized in that** the step of detecting whether the first display device and the second display device are used by a same user through a camera comprises:
acquiring one or more image frames within a coverage area of the camera through the camera, wherein the image frames comprise the first display device, the second display device and the user;
recognizing whether the user in the image frame is the same user according to the image frame.

7. The method according to any one of claims 1-6, **characterized in that** the step of adjusting the image data displayed by the display device with low display priority according to the reference spatial attitude information and the display screen parameters of the display device with low display priority comprises:
extracting a third partial image data from the target 4D image data according to the reference spatial attitude information and the display screen parameters of the display device with low display priority;
updating image data displayed by the display device with low display priority to the third partial image data.

8. The method according to claim 7, **characterized in that** the target 4D image data comprises a plurality of pieces of image data of the target object, and each piece of image data comprises pixel information and spatial position information of corresponding pixel points, wherein the spatial position information is configured to represent a spatial position attribute of the pixel points in a 4D pixel spatial structure of the target object, and the 4D pixel spatial structure corresponds to a real spatial structure of the target object; the step of extracting the third partial image data from the target 4D image data according to the reference spatial attitude information and the display screen parameters of the display device with low display priority comprises:
determining a spatial screening range of the image data according to the reference spatial attitude information and the display screen parameters of the display device with low display priority;
screening out a plurality of pieces of image data belonging to the spatial screening range from the plurality of pieces of image data, wherein the plurality of pieces of image data are the third partial image data.

9. The method according to any one of claims 1-8, **characterized in that** the first display device comprises a virtual reality (VR) display device, and the second display device comprises a personal computer (PC) display device; the target 4D image data comprises image data of a kidney site of the target user; the kidney site comprises kidney, tumor and blood vessel; the kidney has a blood supply relationship with the tumor; the blood vessel comprises a first blood vessel adjacent to the tumor and a second blood vessel connected to the tumor; and the first blood vessel and the second blood vessel are in different positions and have an intersection position; and the method further comprises:
if it is judged that the linkage display function is not enabled, the second blood vessel is displayed on the PC display device while the first blood vessel is displayed on the VR display device;
extracting image data of the intersection position of the first blood vessel and the second blood vessel when a selection instruction for the first blood vessel and the second blood vessel is detected;
and displaying images including the image data of the intersection position on the VR display device and the PC display device respectively.

10. An apparatus for multi-device AI linkage displaying of a VRDS 4D medical image, **characterized in that** the apparatus is applied to a server of a medical imaging apparatus, wherein the medical imaging apparatus comprises the server, a first display device and a second display device; the server is connected to the first display device and the second display device; the apparatus for multi-device AI linkage displaying of VRDS 4D medical image comprises a processing unit and a communication unit, wherein
the processing unit is configured to: acquire a target four-dimensional (4D) image data; extract first partial image data from the target 4D image data according to preset raw spatial attitude information and display screen parameters of the first display device, and extract second partial image data from the target 4D image data according to the raw spatial attitude information and display screen parameters of the second display device, wherein the target 4D image data comprises image data of an inner space and an outer contour of a displayed target object; display the first partial image data on the first display device and the second partial image data on the second display device; and judge whether to enable a linkage display function when receiving first spatial attitude information from the first display device and second spatial attitude information from the second display device, wherein the first spatial attitude information is spatial attitude control information collected by the first display device and the second spatial attitude information is spatial attitude control information collected by the second display device, and the first spatial attitude information is different from the raw spatial attitude information; and if so, select spatial attitude information of a display device with high display priority as reference spatial attitude information; and adjust image data displayed by a display device with low display priority according to the reference spatial attitude information and the display screen parameters of the display device with low display priority; and output linkage prompt information based on the display device with high display priority on the display device with low display priority.

11. The apparatus according to claim 10, **characterized in that** in the aspect of the judging of whether to enable the linkage display function, the processing unit is specifically configured to: detect whether a linkage display switch of the server is turned on; if it is detected that the linkage display switch is turned on, determine to enable the linkage display function; and if detecting that the linkage display switch is turned off, determine not to enable the linkage display function.

12. The apparatus according to claim 10, **characterized in that** in the aspect of the judging of whether to enable the linkage display function, the processing unit is specifically configured to: detect whether a difference between the first spatial attitude information and the second spatial attitude information is within a preset range; if so, determine to enable the linkage display function; if not, determine not to enable the linkage display function.

13. The apparatus according to claim 12, **characterized in that** in the aspect of the detecting of whether a difference between the first spatial attitude information and the second spatial attitude information is within a preset range, the processing unit is specifically configured to: acquire the first spatial attitude information and the second spatial attitude information; calculate the difference between the first spatial attitude information and the second spatial attitude information; compare the difference with the preset range; if the difference falls within the preset range, determine to enable the linkage display function; and if the difference is not within the preset range, determine not to enable the linkage display function.

14. The apparatus according to claim 10, **characterized in that** in the aspect of the judging of whether to enable the linkage display function, the processing unit is specifically configured to: detect whether the first display device and the second display device are used by a same user through a camera; if so, determine to enable the linkage display function; if not, determine not to enable the linkage display function.

15. The apparatus according to claim 14, **characterized in that** in the aspect of the detecting of whether the first display device and the second display device are used by the same user through the camera, the processing unit is specifically configured to: acquire one or more image frames within a coverage area of the camera through the camera, wherein the image frames comprise the first display device, the second display device and the user; recognize whether the user in the image frame is the same user according to the image frame.

16. The apparatus according to any one of claims 10-15, **characterized in that** in the aspect of the adjusting of the image data displayed by the display device with low display priority according to the reference spatial attitude information and the display screen parameters of the display device with low display priority, the processing unit is specifically configured to: extract a third partial image data from the target 4D image data according to the reference spatial attitude information and the display screen parameters of the display device with low display priority; update image data displayed by the display device with low display priority to the third partial image data.

17. The apparatus according to claim 16, **characterized in that** the target 4D image data comprises a plurality of pieces of image data of the target object, and each piece of image data comprises pixel information and spatial position information of corresponding pixel points, wherein the spatial position information is configured to represent a spatial position attribute of the pixel points in a 4D pixel spatial structure of the target object, and the 4D pixel spatial structure corresponds to a real spatial structure of the target object; in the aspect of the extracting of the third partial image data from the target 4D image data according to the reference spatial attitude information and the display screen parameters of the display device with low display priority, the processing unit is specifically configured to: determine a spatial screening range of the image data according to the reference spatial attitude information and the display screen parameters of the display device with low display priority; screen out a plurality of pieces of image data belonging to the spatial screening range from the plurality of pieces of image data, wherein the plurality of pieces of image data are the third partial image data.

18. The apparatus according to any one of claims 10-17, **characterized in that** the first display device comprises a virtual reality (VR) display device, and the second display device comprises a personal computer (PC) display device; the target 4D image data comprises image data of a kidney site of the target user; the kidney site comprises kidney, tumor and blood vessel; the kidney has a blood supply relationship with the tumor; the blood vessel comprises a first blood vessel adjacent to the tumor and a second blood vessel connected to the tumor; and the first blood vessel and the second blood vessel are in different positions and have an intersection position; the processing unit is further specifically configured to: if it is detected that the linkage display function is not enabled, display the second blood vessel on the PC display device while the first blood vessel is displayed on the VR display device; extract image data of the intersection position of the first blood vessel and the second blood vessel when a selection instruction for the first blood vessel and the second blood vessel is detected; and display images including the image data of the intersection position on the VR display device and the PC display device respectively.

19. A medical imaging apparatus, **characterized in that** the apparatus comprises a processor, a memory, a communication interface, and one or more programs, the one or more programs are stored in the memory and configured to be executed by the processor, and the programs comprise instructions for executing the steps in the method according to any one of claims 1-9.

20. A computer readable storage medium, **characterized in that** the computer readable storage medium stores a computer program for electronic data exchange, and wherein the computer program causes a computer to execute the method according to any one of claims 1-9.
